(19) 
**Europäisches Patentamt**
**European Patent Office**
**Office européen des brevets**

(11) **EP 4 001 461 A2**

(12) **EUROPEAN PATENT APPLICATION**

(43) Date of publication:
**25.05.2022 Bulletin 2022/21**

(21) Application number: **21205632.9**

(22) Date of filing: **29.10.2021**

(51) International Patent Classification (IPC):
**C25B 1/02** *(2006.01)*     **C25B 11/052** *(2021.01)*
**C25B 11/085** *(2021.01)*     **C02F 1/461** *(2006.01)*
**H01M 8/16** *(2006.01)*     **C12P 3/00** *(2006.01)*

(52) Cooperative Patent Classification (CPC):
**C25B 11/052; C02F 1/461; C12P 3/00; C25B 1/02;**
**C25B 11/065; C25B 11/085; H01M 8/16**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **24.11.2020 KR 20200159026**

(71) Applicant: **Jeon, Yong Won**
**Seoul 05094 (KR)**

(72) Inventors:
• **JEON, Yong Won**
  **05094 Gwangjin-gu (KR)**
• **KIM, Jun Hyun**
  **Gwangju-si (KR)**
• **KIM, Sung Hyun**
  **Yesan-gun (KR)**

(74) Representative: **Schmitt-Nilson Schraud Waibel**
**Wohlfrom**
**Patentanwälte Partnerschaft mbB**
**Pelkovenstraße 143**
**80992 München (DE)**

(54) **METHOD OF PRODUCING HYDROGEN FROM ALCOHOLIC BEVERAGE FACTORY WASTEWATER USING MICROBIAL ELECTROLYSIS CELL**

(57)    Disclosed is a method of producing hydrogen gas from alcoholic beverage factory wastewater using a single-chamber microbial electrolysis cell (MEC), particularly a method of producing hydrogen capable of increasing hydrogen production yield and hydrogen purity using a microbial electrolysis cell, the method including forming a biofilm on an anode, preparing the pretreated alcoholic beverage factory wastewater, and generating hydrogen by applying an external voltage of 0.8 to 1.2 V.

[Fig. 1]

**EP 4 001 461 A2**

**Description**

BACKGROUND OF THE INVENTION

Field of the Invention

The present invention relates to a method of producing hydrogen from alcoholic beverage factory wastewater using a microbial electrolysis cell, and more particularly to a technique capable of increasing hydrogen production yield and production purity using a microbial electrolysis cell, in which hydrogen is produced by forming a biofilm on an anode and applying an appropriate external voltage to the pretreated alcoholic beverage factory wastewater.

Description of the Related Art

Recently, the regeneration and recycling of resources has received great attention due to the depletion of fossil fuel reserves. In particular, clean and high-efficiency energy sources are being developed around the world in order to solve environmental pollution problems. Among these energy sources, hydrogen is evaluated as an eco-friendly energy source but most hydrogen is ultimately produced by electrolysis using fossil combustion, so efforts to reduce the generation of greenhouse gases that trigger climate change are continuously required.

As part of efforts to minimize the amount of energy input for hydrogen production, a microbial electrolysis cell (MEC) is attracting attention. Briefly explaining the principle of operation of the microbial electrolysis cell, organic material is oxidized in the microbial electrolysis cell, electrons are transferred to an anode, the electrons at the anode are transferred to a cathode when an external voltage is applied to a circuit, and then the electrons at the cathode reduce protons, thereby generating hydrogen gas.

For thermodynamic reasons, a microbial electrolysis cell requires the supply of a small amount of electrical energy in order for the hydrogen production reaction to occur at the cathode, like a water electrolysis cell. Specifically, the microbial electrolysis cell uses electrical energy in a very small amount compared to the water electrolysis cell because wastewater may be used as an additional energy source due to the presence of electroactive microorganisms at the anode. When the substrate for microorganisms is acetate, if an external voltage of only about 0.11 V is applied, it is theoretically possible to produce hydrogen at the same time as wastewater treatment.

However, microbial electrolysis cells developed to date generate methane ($CH_4$) and carbon dioxide ($CO_2$) gases when used for a long time, so performance thereof is remarkably deteriorated, which is undesirable.

[Citation List]

[Patent Literature]

(Patent Document 1) Korean Patent Application Publication No. 10-2019-0071222 (published on June 24, 2019)

SUMMARY OF THE INVENTION

The present invention has been made keeping in mind the problems encountered in the related art, and an object of the present invention is to provide a microbial electrolysis technique in which impurities such as methane or carbon dioxide are not generated even after use for a long time.

Another object of the present invention is to provide a method of producing hydrogen from alcoholic beverage factory wastewater at the same time as purifying the alcoholic beverage factory wastewater.

In order to accomplish the above objects, the present invention provides a method of producing hydrogen using a microbial electrolysis cell including forming a biofilm on the anode of a microbial fuel cell, pretreating alcoholic beverage factory wastewater, converting the microbial fuel cell on which the biofilm is formed into a microbial electrolysis cell, and generating hydrogen by supplying the pretreated alcoholic beverage factory wastewater to the microbial electrolysis cell and applying an external voltage thereto.

The forming the biofilm may include filling the microbial fuel cell with an inoculation solution containing microorganisms, connecting respective ends of a resistor to the anode and the cathode of the microbial fuel cell and measuring a voltage, and replacing the inoculation solution with a new inoculation solution at least once until a constant voltage is established.

The inoculation solution may be prepared by mixing a phosphate buffer saline solution in which sodium acetate is dissolved with activated sludge containing microorganisms at a ratio of 8:2.

The pretreating the alcoholic beverage factory wastewater may include neutralizing the alcoholic beverage factory wastewater to a pH close to 7, subjecting the alcoholic beverage factory wastewater to aeration with nitrogen gas to remove dissolved oxygen, and subjecting the alcoholic beverage factory wastewater to aeration with a sterilizing gas to suppress methanogenesis by methanogens.

The generating the hydrogen may include supplying, as a substrate, a 50 mM PBS solution containing 1 g/L sodium acetate until current and hydrogen gas are stably generated at preset levels and supplying the pretreated alcoholic beverage factory wastewater as a substrate when the generation of current and hydrogen gas is stabilized.

Hydrogen may be produced by applying an external voltage of 0.8 V to 1.2 V to the microbial electrolysis cell.

BRIEF DESCRIPTION OF THE DRAWINGS

The above and other objects, features and other advantages of the present invention will be more clearly understood from the following detailed description taken in conjunction with the accompanying drawings, in which:

FIG. 1 is a flowchart showing a process of producing hydrogen using a microbial electrolysis cell according to an embodiment of the present invention;
FIG. 2 is a graph showing the proportions of gases that are produced at different applied voltages in the microbial electrolysis cell according to an embodiment; and
FIG. 3 is a graph showing the amounts of gases that are produced at different applied voltages in the microbial electrolysis cell according to the embodiment of FIG. 2.

DETAILED DESCRIPTION OF THE INVENTION

Since the present invention may be variously modified and embodied, specific embodiments will be described in detail with reference to the accompanying drawings. However, these embodiments are not to be construed as limiting the present invention, and should be understood to include all modifications, equivalents, and substitutions that fall within the spirit and scope of the present invention.

The present invention pertains to a method of producing hydrogen from alcoholic beverage factory wastewater using a single-chamber microbial electrolysis cell.

As used herein, the term "alcoholic beverage factory wastewater" refers to wastewater remaining after production of alcoholic beverage in a factory producing any one liquor of wine, champagne, liqueur, whiskey, cognac, vodka, tequila, and soju.

Hydrogen production through anaerobic fermentation of microorganisms is a spontaneous reaction, but due to the nature of fermentation, it is difficult to completely oxidize the substrate, so it is common for hydrogen to be produced in an amount smaller than the amount of hydrogen capable of being produced in theory. For example, in theory, a maximum of 4 moles of hydrogen gas may be produced from 1 mole of glucose through a fermentation process, but in reality, not only hydrogen but also various byproducts such as organic acids and the like due to microorganisms are produced based on 1 mole of glucose, so a smaller amount of hydrogen is generated compared to the theoretical amount. In contrast, in the microbial electrolysis cell of the present invention, since electrons are sent to the electrode and then processed similarly to the processing of electrons through oxygen in aerobic decomposition of microorganisms, glucose is completely oxidized into carbon dioxide, and as a result, 12 moles of hydrogen may be produced.

Since the microbial electrolysis cell uses wastewater as both an inoculum and a substrate, various microorganisms including methanogens exist at the anode. Methanogenesis by microorganisms is caused by hydrolysis of acetate used as a substrate, or by coupling hydrogen produced at the cathode with carbon dioxide generated by decomposition of the substrate.

Therefore, in the case in which the reaction of the microbial electrolysis cell is slow and thus the substrate cannot be efficiently decomposed or in which the generated hydrogen is collected slowly, the generation of methane gas increases. Since methanogenesis by microorganisms is not an electrochemical reaction, it may occur throughout the reactor.

Particularly, in the microbial electrolysis cell using wastewater as a substrate, microorganisms responsible for decomposing the substrate and sending electrons to the anode form a colony on the surface of the anode. Here, the composition of the colony may be changed by the microorganisms contained in the wastewater. As a result of the change in composition, the electrical activity of the microbial colony is inhibited at the anode, so the hydrogen production yield and production rate of the microbial electrolysis cell decrease, and the substrate decomposition rate is also slow.

In addition, methanogens that may be present in wastewater may proliferate in large amounts and generate methane gas using the substrate or using the produced hydrogen. The methane gas thus generated further inhibits the production of hydrogen in the microbial electrolysis cell.

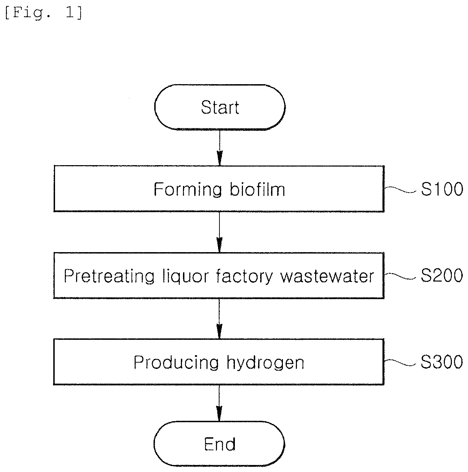

FIG. 1 is a flowchart showing the process of producing hydrogen using a microbial electrolysis cell according to an embodiment.

The method of producing hydrogen according to an embodiment includes forming a biofilm (100), pretreating alcoholic beverage factory wastewater (200), and producing hydrogen (300).

(a) Forming biofilm (100)

Before supplying alcoholic beverage factory wastewater as a substrate to the microbial electrolysis cell, an electroactive microbial colony biofilm is first formed on the anode. The microbial electrolysis cell of the present invention operates as a microbial fuel cell using an air cathode in order to form a biofilm. For operation of the fuel cell, a mixture of a 50 mM PBS solution containing 1 g/L sodium acetate with activated sludge previously collected from a sewage treatment plant is supplied until a constant reference voltage is established across an external resistance of 1 kΩ.

(b) Pretreating alcoholic beverage factory wastewater (200)

Wastewater collected from alcoholic beverage factories is typically strongly basic and thus has to be neutralized to a pH of about 7. Moreover, alcoholic beverage factory wastewater has to be aerated with nitrogen gas for a predetermined period of time in order to remove dissolved oxygen therefrom, and also with a sterilizing gas such as acetylene gas for a predetermined period of time in order to suppress methanogenesis by methanogens.

(c) Producing hydrogen (300)

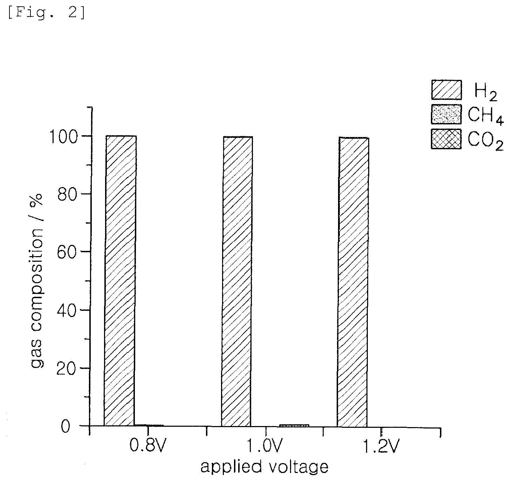

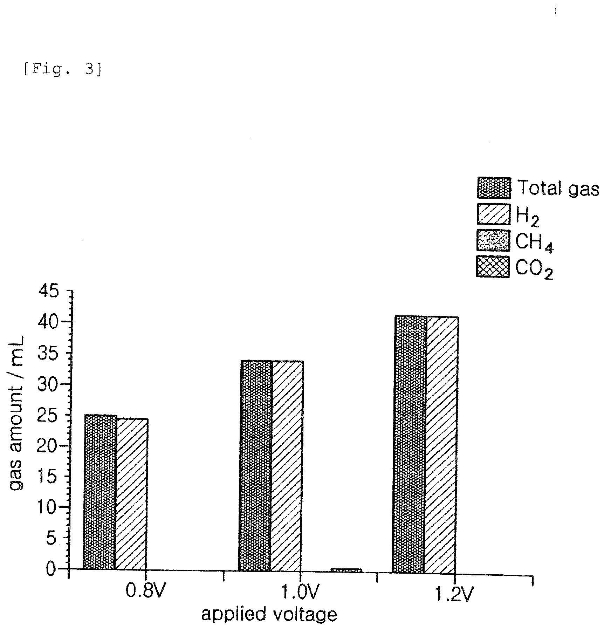

When the reference voltage is established, the air cathode of the microbial fuel cell is converted into a cathode having a proton reduction catalyst, and an external voltage is applied thereto, so the microbial fuel cell operates as a microbial electrolysis cell. Then, a 50 mM PBS solution containing 1 g/L sodium acetate is supplied as a substrate until electricity and hydrogen gas are stably generated in the state in which the external voltage is applied.
When the generation of electricity and hydrogen gas is stabilized at a preset level, the alcoholic beverage factory wastewater prepared above is supplied thereto.
Below is a detailed description of the production of hydrogen from soju factory wastewater according to an exemplary embodiment.
For reference, FIG. 2 is a graph showing the proportions of gases that are produced at different applied voltages in the microbial electrolysis cell of the present embodiment, and FIG. 3 is a graph showing the amounts of gases that are produced at different applied voltages in the microbial electrolysis cell according to the embodiment of FIG. 2.

[Example]

1. Configuration of microbial electrolysis cell (MEC)

The microbial electrolysis cell has a shape of a horizontal cylinder having a length of 5 cm and a diameter of 3 cm with a capacity of 24 mL. The anode is a carbon fiber brush having a length of 2.5 cm and a diameter of 2.5 cm, particularly a cylindrical brush in which carbon fiber is fixed by twisting stainless steel wire, and is located at one end of the cylinder. The carbon fiber brush electrode exhibits an increased specific surface area so as to enable adhesion and growth of a large number of electroactive microorganisms. For the anode, a hole was made in an acrylic plate and the stainless-steel wire having carbon fiber fixed thereto was inserted into the hole and fixed with an epoxy resin.
For the cathode located at the end of the cylinder opposite the anode, an air cathode, in which a Pt/C catalyst was fixed on one surface of carbon cloth using a Nafion binder and 4 layers of air diffusion were formed with a Teflon solution on the remaining surface thereof, was used. The apparent surface area of the cathode was 7.07 cm$^2$. The cathode was fixed at a position opposite the anode of the cylinder with a plate having a hole having a diameter of 2.5 cm in the center thereof and an O-ring so as to prevent leakage of the electrolyte and enable the inflow of outdoor air through the electrode during the initial period of forming a constant voltage.
During operation of the microbial electrolysis cell, the plate having a hole was replaced with an acrylic plate having no hole in order to block the inflow of outdoor air. At the top of the cell, a small cylinder, which was sealed with a rubber stopper to collect the generated gas, was fixed in the manner of a screw cap so that gas collection and substrate exchange were possible.

2. Microbial inoculation of microbial electrolysis cell and pretreatment of soju wastewater

Activated sludge was collected from a sewage treatment plant configured to treat domestic sewage, as a microbial inoculum for the microbial electrolysis cell, and was then stored in a refrigerator at 4°C. Thereafter, an inoculation solution was prepared by mixing the collected activated sludge with a 50 mM phosphate buffer saline solution in which sodium acetate was dissolved at a concentration of 1 g/L at a ratio of 2:8.

In order to form a biofilm on the anode, the inoculation solution prepared as described above was placed in the microbial electrolysis cell, and a 1 kΩ resistor was connected at respective ends to the anode and to the cathode using an external conductor. The inoculation solution was exchanged periodically until a predetermined level of reference voltage was established.

Since the soju wastewater collected from the water treatment plant of the soju factory had a basic pH of 11 to 12.51, the pH thereof was changed to a neutral pH of 7 by gradually adding 50 mM of sodium phosphate monobasic and sodium phosphate dibasic to the soju wastewater.

All solutions placed in the microbial electrolysis cell were aerated with nitrogen gas for 10 minutes in order to remove dissolved oxygen therefrom and with 10% acetylene gas for 10 minutes in order to suppress methanogenesis by methanogens.

3. Operation of microbial electrolysis cell and capture of hydrogen

As described above, a 1 kΩ resistor was connected at respective ends to the two electrodes of the microbial electrolysis cell, and the inoculation solution was supplied batchwise until a constant voltage was established in the cell. After a predetermined reference voltage was established, the acrylic plate having a hole, which fixes the cathode, was replaced with an acrylic plate having no hole in order to block the inflow of outdoor air.

When a biofilm was formed on the anode, the microbial fuel cell was converted into a microbial electrolysis cell, and a constant voltage was applied to the cell by connecting the microbial electrolysis cell to a power supply. Then, a 50 mM PBS solution containing 1 g/L sodium acetate was supplied as a substrate until electricity and hydrogen gas were stably generated.

When electricity and hydrogen gas were stably generated, the pretreated soju wastewater was introduced, and then the output current was measured. Moreover, hydrogen produced using the microbial electrolysis cell was captured through a silicone tube by performing water displacement by inserting a measuring cylinder filled with water upside down into a water tank filled with water.

[Experimental result]

1. Analysis of produced gas and performance analysis of microbial electrolysis cell

The amount of gas that was produced and captured was determined using the volume captured in the measuring cylinder, and the volume of each gas was calculated by multiplying the total gas volume by the proportion of each component using gas chromatography.

The performance of the microbial electrolysis cell was determined by calculating the coulombic efficiency (CE), cathodic hydrogen recovery (CHR), overall hydrogen recovery (OHR), hydrogen production rate (HPR), and hydrogen yield ($Y_{H2}$), and the calculated results were compared at different applied voltages.

The coulombic efficiency is the ratio of the theoretical electric charge from the added substrate to the actual electric charge flowing through the external conductor, showing how much of the substrate was converted into the current. The coulombic efficiency was calculated using [Equation 1] below.

[Equation 1]

$$CE = \frac{\int_{t=0}^{t} I \, dt}{12.06 \, v_{cell} \, COD}$$

Here, I is the magnitude of the electric charge flowing over time when integrated with the amount of time for which current flows. $v_{cell}$ and COD are the volume of the substrate placed in the cell and chemical oxygen demand, respectively. 12.06 is the magnitude of the electric charge required to reduce 1 mg of oxygen into water.

The cathodic hydrogen recovery is the ratio of the calculated number of moles of hydrogen to the number of moles of hydrogen captured at the measured current, showing how much hydrogen was generated at the measured current. The cathodic hydrogen recovery was calculated using [Equation 2] below.

[Equation 2]

$$CHR = \frac{v_{H_2} P2F}{RT \int_{t=0}^{t} I dt}$$

Here, $v_{H_2}$ is the volume of captured hydrogen, P is the pressure of captured gas, F is the Faraday constant, R is the gas constant, and T is the gas temperature relative to absolute temperature.

The overall hydrogen recovery is the ratio of the number of moles of theoretically produced hydrogen to the number of moles of hydrogen actually recovered from the added substrate, and was calculated as the product of the coulombic efficiency and the cathodic hydrogen recovery.

The hydrogen production rate is a value showing how much hydrogen ($m^3$) is produced per unit time (days) in a microbial electrolysis cell per unit volume ($m^3$), and was calculated using [Equation 3] below.

[Equation 3]

$$HPR = \frac{43.2 (I/v_{cell}) CHR}{F c_g (T)}$$

Here, 43.2 is the unit conversion factor, and $c_g(T)$ is the gas concentration at temperature T.

2. Result of hydrogen production in microbial electrolysis cell at different applied voltages

When soju factory wastewater was used as a substrate for the microbial electrolysis cell, there was a difference in the amounts of gases that were produced depending on the applied voltage, but almost no methane gas or carbon dioxide gas was contained in the gas produced and captured using the microbial electrolysis cell, and the total produced gas was confirmed to be composed of at least 99% hydrogen gas at all applied voltages. In particular, only hydrogen was detected at the applied voltage of 1.2 V (FIG. 2 and Table 1).

[Table 1]

| Applied voltage | Hydrogen (%) | Methane (%) | Carbon dioxide (%) |
|---|---|---|---|
| 0.8 V | 99.6 | 0.4 | 0 |
| 1.0 V | 99.2 | 0 | 0.83 |
| 1.2 V | 100 | 0 | 0 |

At the applied voltages of 0.8 V, 1.0 V, and 1.2 V, the total gas amounts produced by the microbial electrolysis cell were 23.8 mL, 34.3 mL, and 41.5 mL, respectively (FIG. 3 and Table 2).

[Table 2]

| Applied voltage | Total amount of gas produced (mL) | Hydrogen (mL) | Methane (mL) | Carbon dioxide (mL) |
|---|---|---|---|---|
| 0.8 V | 23.8 | 23.6 | 0.112 | 0 |
| 1.0 V | 34.3 | 34.0 | 0 | 0.278 |

(continued)

| Applied voltage | Total amount of gas produced (mL) | Hydrogen (mL) | Methane (mL) | Carbon dioxide (mL) |
|---|---|---|---|---|
| 1.2 V | 41.5 | 41.5 | 0 | 0 |

Meanwhile, when hydrogen was no longer generated at the applied voltage, a 50 mM phosphate buffer saline solution in which sodium acetate was dissolved at a concentration of 1 g/L was supplied again to restore a constant current, after which the alcoholic beverage factory wastewater was supplied again, showing that hydrogen can be produced continuously.

As the supply of alcoholic beverage factory wastewater is repeated, the microbial colony is affected and the shape of the current curve obtained for the corresponding batch changes, and when hydrogen is not generated, a 50 mM PBS solution containing 1 g/L sodium acetate is supplied to the cell to restore the shape of the current curve, followed by supply of the alcoholic beverage factory wastewater again, whereby hydrogen gas can be continuously generated. In contrast, in the case in which the performance of the microbial electrolysis cell deteriorates and thus the generation of hydrogen decreases, the production of impurities such as methane increases, and the purity decreases.

As is apparent from the above description, the method of producing hydrogen according to an embodiment of the present invention enables hydrogen to be produced for a long time with high purity and high yield by minimizing the generation of methane gas and carbon dioxide gas.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1.  A method of producing hydrogen from alcoholic beverage factory wastewater using a microbial electrolysis cell, comprising:

    forming a biofilm on an anode of a microbial fuel cell;
    pretreating alcoholic beverage factory wastewater;
    converting the microbial fuel cell on which the biofilm is formed into a microbial electrolysis cell; and
    generating hydrogen by supplying the pretreated alcoholic beverage factory wastewater to the microbial electrolysis cell and applying an external voltage thereto.

2.  The method of claim 1, wherein the forming the biofilm comprises:

    filling the microbial fuel cell with an inoculation solution containing microorganisms,
    connecting ends of a resistor to an anode and a cathode of the microbial fuel cell and measuring a voltage, and
    replacing the inoculation solution with a new inoculation solution at least once until a constant voltage is established.

3.  The method of claim 2, wherein the inoculation solution is prepared by mixing a phosphate buffer saline solution in which sodium acetate is dissolved with activated sludge containing microorganisms at a ratio of 8:2.

4.  The method of any of the preceding claims, wherein the pretreating the alcoholic beverage factory wastewater comprises:

    neutralizing the alcoholic beverage factory wastewater to a pH close to 7,
    subjecting the alcoholic beverage factory wastewater to aeration with nitrogen gas to remove dissolved oxygen, and
    subjecting the alcoholic beverage factory wastewater to aeration with a sterilizing gas to suppress methanogenesis by a methanogen.

5.  The method of any of the preceding claims, wherein the generating the hydrogen comprises:

    supplying as a substrate a 50 mM PBS solution containing 1 g/L sodium acetate until current and hydrogen gas are stably generated at preset levels, and

supplying the pretreated alcoholic beverage factory wastewater as a substrate when generation of current and hydrogen gas is stabilized.

6. The method of any of the preceding claims, wherein hydrogen is produced by applying an external voltage of 0.8 V to 1.2 V to the microbial electrolysis cell.

[Fig. 1]

[Fig. 2]

[Fig. 3]

**EP 4 001 461 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190071222